# EUROPEAN PATENT APPLICATION

(11) **EP 2 275 768 A2**
(43) Date of publication of application: **19.01.2011**
(21) Application number: 10167115.4
(22) Date of filing: 24.06.2010
(51) Int. Cl.: F41A 33/06, F41B 11/34, F41B 11/06

(54) **Toy gun with recoil mechanism**

(30) Priority: 14.07.2009 TW 098212772
(71) Applicant: Yih Kai Enterprise Co., Ltd, Tainan County 71082 (TW)
(72) Inventor: Hu, Shih-Che, 71082, Tainan County (TW)
(74) Representative: Metz, Paul

(57) **Abstract**

A gun-lock assembly **(A)** of a toy gun is disclosed to have a gasket ring **(7)** mounted on the periphery of a piston **(2)** near its rear end and peripherally stopped at the peripheral wall of an accommodation chamber **(11)** of the gun-lock housing **(1) .** When an intake flow of gas is guided into the piston **(2),** the gasket ring **(7)** is moved with the piston **(2)** relative to the housing **(1)** to extend an airtight space in the accommodation chamber **(11)** for receiving the intake flow of gas to enhance the recoil of the toy gun.

## Description

### BACKGROUND OF THE INVENTION

### 1. Field of the Invention

The present invention relates to gun lock technology and more particularly, to a gun-lock assembly of a toy gun, which greatly enhances the recoil of the toy gun.

### 2. Description of the Related Art

Regular toy air-soft guns are designed like a real gun. These toy air-soft guns have the same outer appearance of a real gun. They operate in the same manner as a real gun. Further, similar to a real gun, these toy air-soft guns produce a recoil upon shooting.

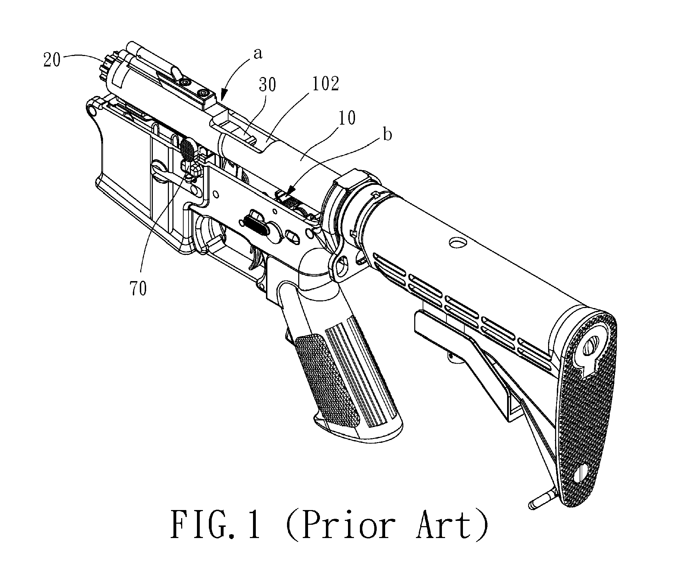

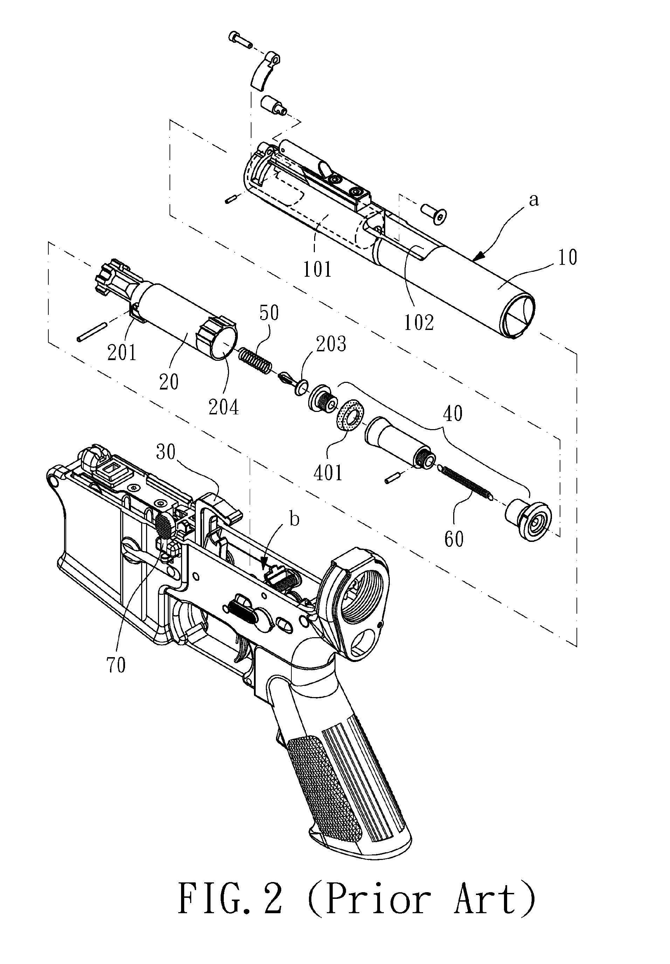

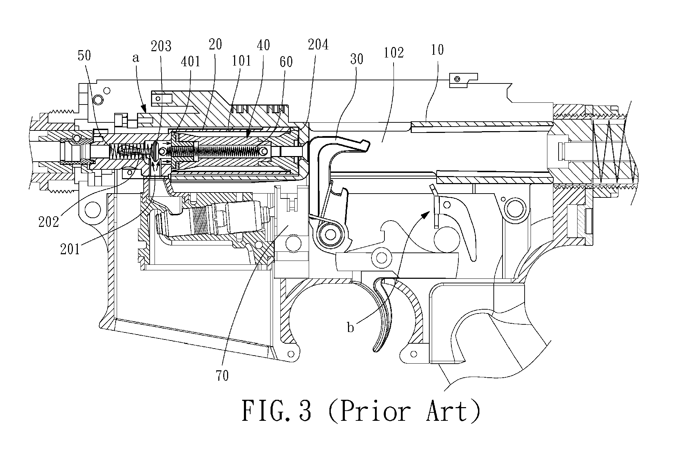

Further, the strength of the firing force and recoil of a toy air-soft gun has a greatly concern with the design of the gun-lock assembly. FIGS. 1 and 2 show a gun-lock assembly **a** of a toy air-soft gun according to the prior art. According to this design, the gun-lock assembly **a** comprises a housing **10** and a piston **20.** The housing **10** comprises an accommodation chamber **101** (see FIGS. 2 and 3) for accommodating the piston **20,** and opening **102** transversely extending across the middle part at a rear side relative to the accommodation chamber **101** within which a hammer **30** of a firing control mechanism **b** is moved (see FIGS. 1 and 3), and an end block **40** affixed to the rear end of the accommodation chamber **101.** The end block **40** has a gasket ring (rubber ring) **401** mounted thereon.

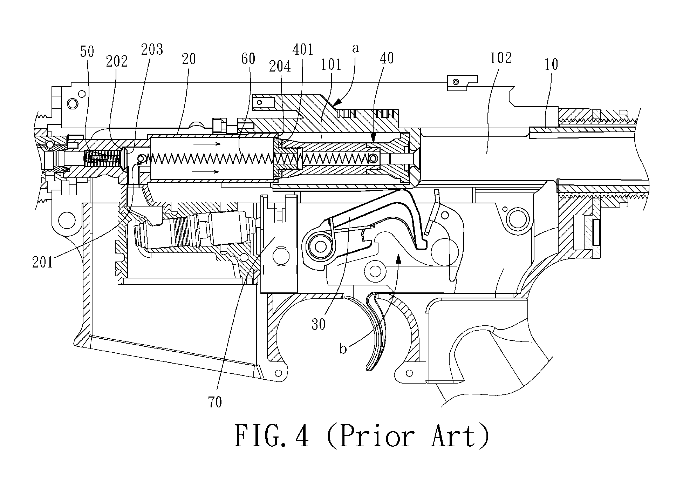

The piston **20** is inserted through the front end of the housing **10** into the inside of the accommodation chamber **101.** The piston **20** has a stepped axial hole **202** axially extending through the front and rear ends thereof, a radial gas inlet **201** located on the periphery in communication with the axial hole **202** (see FIG.3). Further, a first spring member **50** mounted in the stepped axial hole **202** near the front end of the piston **20,** and a stopper bolt **203** is supported on the first spring member **50** axially movable in the stepped axial hole **202** between two positions to close/open the stepped axial hole **202.** Further, a second spring member **60** is mounted in the stepped axial hole **202** of the piston **20** and affixed with its front end to the inside wall of the piston **20** and its rear end to the end block **40.** By means of the arrangement of the second spring member **60,** the piston **20** is axially movable in the accommodation chamber **101** of the housing **10** (see FIGS. 3 and 4). Further, the piston **20** has a rear hole **204** on the rear end. Normally, the piston **20** is not moved in the accommodation chamber **101** of the housing **10,** and the spring force of the second spring member **60** causes the rear hole **204** of the piston **20** to be stopped by the end block **40** at the rear end of the accommodation chamber **101** (see FIG. 3).

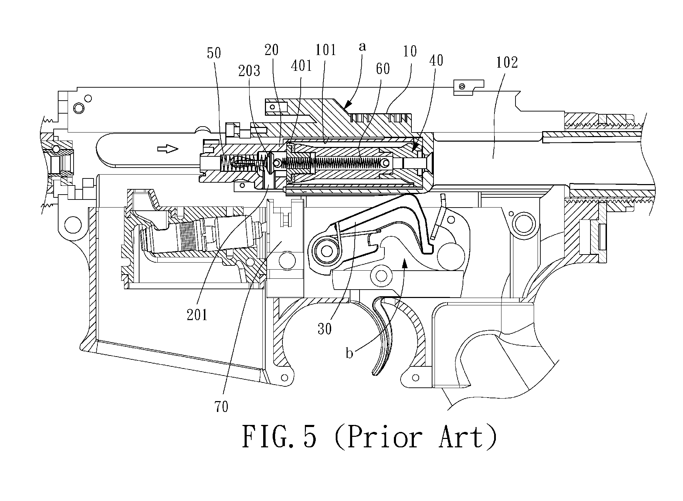

When a user operated the firing control mechanism **b** to strike the hammer **30,** a flow of gas is discharged from a gas supply unit **70** through the gas inlet **201** into the stepped axial hole **202** of the piston **20.** At this time, the intake flow of gas flows partially toward the front end of the piston **20** and partially toward the rear end of the piston **20** (see FIG. 4). The forward flowing of the intake flow of gas forces forwards the stopper bolt **203** forwards to close the stepped axial hole **202** (see FIG. 4). Thereafter, all the intake flow of gas flows toward the rear end of the piston **20.** At this time, the gasket ring **401** is stopped against the inside wall of the piston **20,** keeping the piston **20** in an airtight status, and the continuous flow of gas imparts a pressure to the end block **40** and the housing **10** against the piston **20,** thereby causing the housing **10** to be moved backwards through a predetermined distance, and therefore a recoil is produced. When the supply of gas is stopped, the housing **10** is not moved backwards further, and the second spring member **60** returns the piston **20** to its former position (see FIG. 5).

As stated above, when a flow of gas enters the piston **20** to impart a pressure to the end block **40,** a recoil is produced. However, due to limited airtight space behind the end block **40,** the strength of the recoil produced upon a backward displacement of the housing **10** is limited, lowering the lifelike effect. Therefore, an improvement is this regard is necessary.

### SUMMARY OF THE INVENTION

The present invention has been accomplished under the circumstances in view. It is one object of the present invention to provide a gun-lock assembly for toy gun, which enhances the recoil of the toy gun.

To achieve this and other objects of the present invention, a gun-lock assembly comprises a housing, a piston, a first spring member, a stopper bolt, a second spring member and a gasket ring. The housing comprises an accommodation chamber longitudinally extending through front and rear ends thereof, an opening transversely extending across a middle part thereof at a rear side relative to the accommodation chamber for allowing movement of a hammer of a firing control mechanism, and an end block affixed to the rear end thereof. The piston is inserted through the front end of the housing into the accommodation chamber, comprising a stepped axial hole axially extending through front and rear ends thereof and a radial gas inlet located on the periphery thereof in communication with the stepped axial hole. The first spring member is mounted in the stepped axial hole near the front end of the piston. The stopper bolt is supported on the first spring member and movable to close the front end of the piston upon a supply of a forced flow of gas through the radial gas inlet into the stepped axial hole. The second spring member is mounted in the stepped axial hole and connected between the inside wall of the piston and the end block. The gasket ring is mounted on the periphery of the piston near the rear end of the piston and peripherally stopped at the peripheral wall of the accommodation chamber of the housing. The gasket ring is movable with the piston relative to the housing to extend an airtight space in the accommodation chamber for receiving a supply of a forced flow of gas, thereby enhancing the recoil of the toy gun.

Further, the piston has a rear hole on the rear end thereof. The rear hole of the piston is stopped by the end block before the supply of a flow of gas through the radial gas inlet into the stepped axial hole.

### BRIEF DESCRIPTION OF THE DRAWINGS

FIG. 1 is an elevational view of a gun-lock assembly in a toy gun according to the prior art.
FIG. 2 is an exploded view of the gun-lock assembly shown in FIG. 1.
FIG. 3 is a plain view of the gun-lock assembly shown in FIG. 1.
FIG. 4 is a schematic drawing showing the prior art gun-lock assembly in action (I).
FIG. 5 is a schematic drawing showing the prior art gun-lock assembly in action (II).
FIG. 6 is an exploded view of a gun-lock assembly of a toy gun in accordance with the present invention.
FIG. 7 is a plain assembly view of FIG. 6.
FIG. 8 is a schematic drawing showing the gun-lock assembly of the present invention in action (I).
FIG. 9 is a schematic drawing showing the gun-lock assembly of the present invention in action (II).

### DETAILED DESCRIPTION OF THE PREFERRED EMBODIMENT

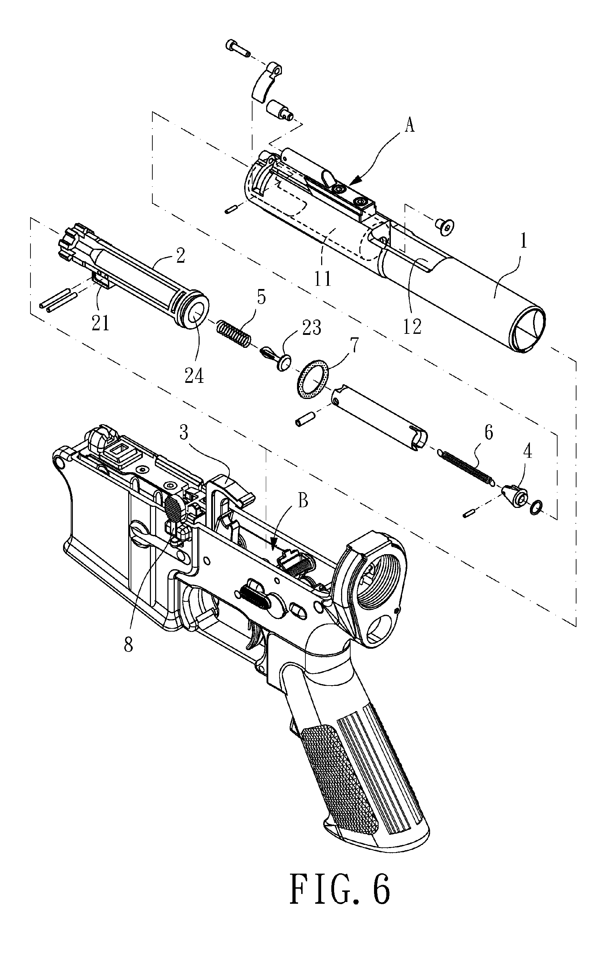

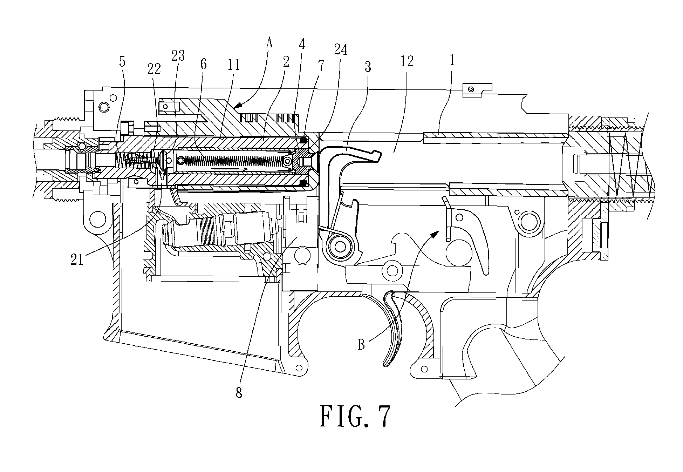

Referring to FIGS. 6 and 7, a gun-lock assembly **A** of a toy gun is shown comprising a housing **1** and a piston **2.**

The housing **1** has an accommodation chamber **11** defined therein and longitudinally extending through front and rear ends thereof, an opening **12** transversely extending across the middle part thereof behind the accommodation chamber **11** within which a hammer **3** of a firing control mechanism **B** is moved (see FIG. 7), and an end block **4** affixed to the rear end of the accommodation chamber **11.**

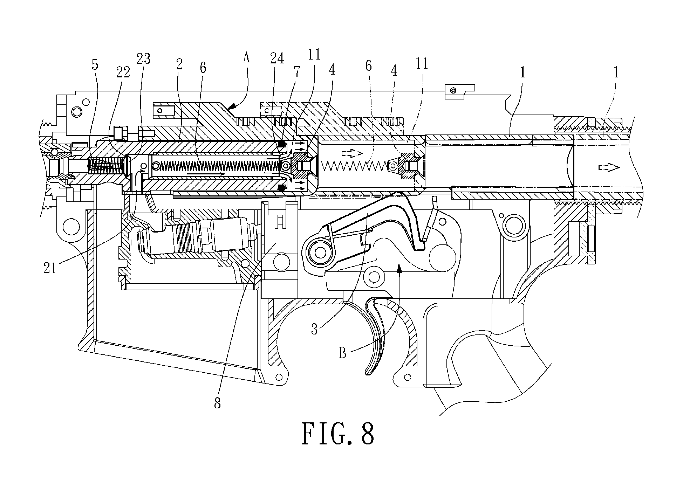

The piston **2** is inserted through the front end of the housing **1** into the inside of the accommodation chamber **11.** The piston **2** has a stepped axial hole **22** axially extending through the front and rear ends thereof, a radial gas inlet **21** located on the periphery in communication with the axial hole **22.** Further, a first spring member **5** mounted in the stepped axial hole **22** near the front end of the piston **2,** and a stopper bolt **23** is supported on the first spring member **5** axially movable in the stepped axial hole **22** between two positions to close/open the stepped axial hole **22.** Normally, the stopper bolt **23** is supported on the first spring member **5** in the open position where the stepped axial hole **22** is opened. When a flow of gas enters the radial gas inlet **21,** the stopper bolt **23** is forced forwards by the pressure of the intake flow of gas to compress the first spring member **5** and to further close the stepped axial hole **22** (see FIG. 8). Further, a second spring member **6** is mounted in the stepped axial hole **22** of the piston **2** and affixed with its front end to the inside wall of the piston **2** and its rear end to the end block **4.** By means of the arrangement of the second spring member **6,** the piston **2** is axially movable in the accommodation chamber **11** of the housing **1.** Further, a gasket ring **7** is mounted on the periphery of the piston **2** near its rear end and stopped at the peripheral wall of the accommodation chamber **11** of the housing **1** (see FIGS. 7 and 8).

Further, the piston **2** has a rear hole **24** on the rear end. Normally, the piston **2** is not moved in the accommodation chamber **11** of the housing **1,** and the spring force of the second spring member **6** causes the rear hole **24** of the piston **2** to be stopped by the end block **4** at the rear end of the accommodation chamber **11** (see FIG. 7).

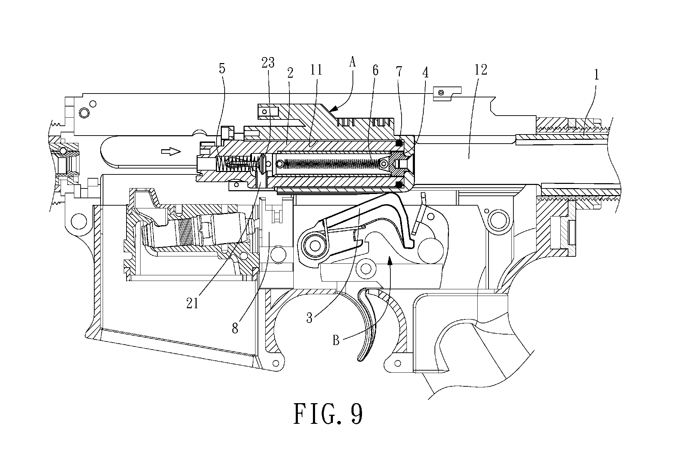

When a user operated the firing control mechanism **B** to strike the hammer **3,** a flow of gas is discharged from a gas supply unit **8** through the gas inlet **21** into the stepped axial hole **22** of the piston **2.** At this time, the intake flow of gas flows partially toward the front end of the piston **2** and partially toward the rear end of the piston **2** (see FIG. 7). The forward flowing of the intake flow of gas forces forwards the stopper bolt **23** forwards to close the stepped axial hole **22** (see FIG. 8). Thereafter, all the intake flow of gas flows toward the rear end of the piston **2** to impart a pressure to the end block **4** and the housing **1** against the piston **2,** thereby opening the rear hole **24** of the piston **2** from the end block **4** for the passing of the intake flow of gas to the inside of the accommodation chamber **11** of the housing **1** (see FIG. 8). At this time, the airtight space in the accommodation chamber **11** is extended. Subject to the equation of Force(F)=Pressure(P) x Area(A), the invention greatly enhances the recoil of the toy gun when compared to the prior art design. After a predetermined backward displacement of the housing **1,** the supply of gas is stopped, and the second spring member **6** returns to its former shape, thereby returning the piston **2** to its former position (see FIG. 9).

As stated above, when the intake flow of gas wholly moves toward the rear end of the piston **2,** the gasket ring **7** is moved with the piston **2** relative to the housing **1** for allowing the intake flow of gas to flow through the rear hole **24** of the piston **2** into the inside of the accommodation chamber **11** of the housing **1** to extend the area of the accommodation chamber **11.** By means of extending the area of the accommodation chamber **11,** the recoil of the toy gun is relatively enhanced.

A prototype of gun-lock assembly has been constructed with the features of FIGS. 6∼9. The gun-lock assembly functions smoothly to provide all of the features disclosed earlier.

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A gun-lock assembly **(A),** comprising:
a housing **(1),** said housing **(1)** comprising an accommodation chamber **(11)** longitudinally extending through front and rear ends thereof, an opening **(12)** transversely extending across a middle part thereof at a rear side relative to said accommodation chamber **(11)** for allowing movement of a hammer **(3)** of a firing control mechanism **(B),** and an end block **(4)** affixed to the rear end thereof;
a piston **(2)** inserted through the front end of said housing **(1)** into said accommodation chamber **(11),** said piston **(2)** comprising a stepped axial hole **(22)** axially extending through front and rear ends thereof and a radial gas inlet **(21)** located on the periphery thereof in communication with said stepped axial hole **(22);**
a first spring member **(5)** mounted in said stepped axial hole **(22)** near the front end of said piston **(2);**
a stopper bolt **(23)** supported on said first spring member **(5)** and movable to close the front end of said piston **(2)** upon a supply of a forced flow of gas through said radial gas inlet **(21)** into said stepped axial hole **(22);**
a second spring member **(6)** mounted in said stepped axial hole **(22)** and connected between an inside wall of said piston **(2)** and said end block **(4);**
wherein a gasket ring **(7)** is mounted on the periphery of said piston **(2)** near the rear end of said piston **(2)** and peripherally stopped at the peripheral wall of said accommodation chamber **(11)** of said housing **(1),** said gasket ring **(7)** being movable with said piston **(2)** relative to said housing **(1)** to extend an airtight space in said accommodation chamber **(11)** for receiving a supply of a forced flow of gas.

2. The gun-lock assembly **(A)** as claimed in claim 1, wherein said piston **(2)** has a rear hole **(24)** on the rear end thereof, said rear hole **(24)** being stopped by said end block **(4)** before the supply of a flow of gas through said radial gas inlet **(21)** into said stepped axial hole **(22).**
